(19) **Europäisches Patentamt / European Patent Office / Office européen des brevets**

(11) **EP 1 265 529 B1**

(12) **FASCICULE DE BREVET EUROPEEN**

(45) Date de publication et mention
de la délivrance du brevet:
**16.06.2010 Bulletin 2010/24**

(21) Numéro de dépôt: **01911815.7**

(22) Date de dépôt: **01.03.2001**

(51) Int Cl.:
***A61B 5/103*** (2006.01)

(86) Numéro de dépôt international:
**PCT/FR2001/000605**

(87) Numéro de publication internationale:
**WO 2001/072222 (04.10.2001 Gazette 2001/40)**

(54) **MESURE NON INVASIVE DU TAUX DE BILIRUBINE DE LA PEAU**

NICHTINVASIVE MESSUNG DER HAUTBILIRUBINRATE

NON-INVASIVE MEASUREMENT OF SKIN BILIRUBIN LEVEL

(84) Etats contractants désignés:
**AT BE CH CY DE DK ES FI FR GB GR IE IT LI LU MC NL PT SE TR**

(30) Priorité: **24.03.2000 FR 0003777**

(43) Date de publication de la demande:
**18.12.2002 Bulletin 2002/51**

(73) Titulaire: **MED&LED**
**6060 Gilly (BE)**

(72) Inventeur: **DICK, Jean-Michel**
**95480 Pierrelaye (FR)**

(74) Mandataire: **Schmit, Christian Norbert Marie et al**
**SCHMIT CHRETIEN**
**16, rue de la Paix**
**75002 Paris (FR)**

(56) Documents cités:
**EP-A- 0 747 002      DE-A- 3 827 457**
**US-A- 4 398 541      US-A- 5 259 382**
**US-A- 5 645 061**

**Description**

La présente invention se rapporte à un procédé et à un dispositif d'analyse non invasive d'un tissu, par exemple de la peau.

De nombreux travaux ont montré qu'il était possible en envoyant de la lumière blanche sur un tissu, tel par exemple que la peau ou le tissu d'une plante, d'obtenir en analysant la lumière réfléchie des informations concernant la nature du tissu et notamment la concentration des différents éléments qui le constituent. Le principe général est que les composantes de longueurs d'onde diverses qui composent la lumière blanche sont réfléchies de façon différente selon les constituants que la lumière rencontre. Par une analyse fine et continue de la lumière réfléchie, on conçoit qu'il est possible d'obtenir une analyse non invasive assez précise du tissu examiné.

Les dispositifs connus qui fonctionnent sur ce principe nécessitent cependant un appareillage coûteux d'analyse optique tel que des spectrographes et des calculateurs puissants d'analyse des données recueillies ainsi qu'un étalonnage délicat des appareils.

Le document US 5 645 061 décrit un procédé de détermination des variations de dimensions de particules de tissus de manière non traumatisante. La comparaison de spectres de réflexion permet la mesure de l'hémoglobine et de son taux d'oxygénation.

L'invention propose un procédé et un dispositif de mesure permettant de s'affranchir de ces difficultés de mise en oeuvre.

À cet effet, le procédé de mesure du taux d'un constituant d'un tissu, en particulier du taux de bilirubine dans la peau, se caractérise selon l'invention telle que définie dans les revendications 1 à 4.

Un dispositif conforme à l'invention est défini dans les revendications 5 à 11.

L'invention et sa mise en oeuvre apparaîtront plus clairement à l'aide de la description qui va suivre faite en référence aux dessins annexés dans lesquels :

la figure 1, explique le principe de la réflexion optique de la lumière par la peau.

la figure 2 montre trois courbes de LIR enregistrés pour trois sujets, ces courbes ayant subi un traitement mathématique pour les mettre au même niveau pour un seuil de longueur d'onde déterminé ;

la figure 3 montre de façon schématique le principe d'un appareil conçu selon l'invention ;

les figures 4, 5 et 6 montrent le principe d'utilisation pratique de l'appareil ;

la figure 7 montre, de façon schématique, une variante de réalisation d'un appareil de mesure conforme à l'invention.

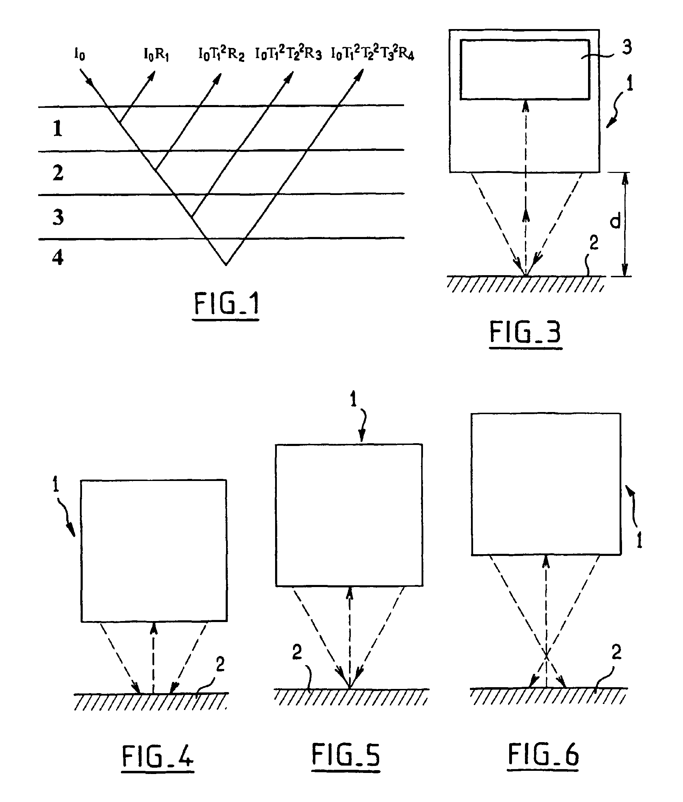

En se reportant tout d'abord à la figure 1, on va rappeler le principe de la propagation de la lumière et de sa réflexion sur la peau. Ce principe est notamment explicité dans la publication de Dawson, Barker et autres : « une étude théorique et expérimentale de l'absorption et de la diffusion de la lumière par la peau in vivo » (Phys. Med. Biol. 1980, vol. 25, n° 4, pages 695 -- 709).

En considérant dans l'ordre que la couche 1 est la couche cornée, que la couche 2 correspond à l'épiderme, que la couche 3 correspond au derme et que la couche 4 correspond à l'hypoderme, si l'on appelle respectivement R1, R2, R3 et R4 le facteur de réflexion des couches successives et T1, T2, T3 et T4 le facteur de transmission des mêmes couches, la lumière réfléchie globalement est de la forme :

$$I = I_0 R_1 + I_0 T_1^2 R_2 + I_0 T_1^2 T_2^2 R_3 + I_0 T_1^2 T_2^2 T_3^2 R_4 ..$$

Si la peau n'est pas trop sèche, les facteurs de réflexion R1, R2 et R3 sont nettement inférieurs à R4; alors la réflexion globale se réduit à :

$$R = (I/I_0) \cong T_1^2 T_2^2 T_3^2 R_4 ..$$

En prenant le Logarithme Népérien (LN) de l'inverse de la réflexion, qu'on appelle le LIR, on obtient :

$$LIR = -LN(T_1^2) - LN.(T_2^2) - LN(T_3^2) - LN(R_4)$$

Ce LIR, représenté pour toutes les longueurs d'onde, permet de mettre en évidence les caractéristiques de la peau. On peut ainsi tracer des courbes et en déduire les qualités de la peau en analysant les bandes d'absorption concernées qui sont caractéristiques des divers constituants de la peau, et en déduire par exemple la teneur en bilirubine, en hémoglobine, en mélanine, etc..

Comme déjà explicité, la mise en pratique de cette théorie se heurte à de grosses difficultés d'appareillage et de calibrage, les courbes variant considérablement d'un sujet à l'autre, en particulier en fonction de la pigmentation propre du sujet.

Conformément à l'invention, on a néanmoins constaté qu'il était possible de s'affranchir des difficultés liées à des réactions très différentes de la peau d'un sujet à un autre en « calant » toutes les courbes au niveau d'un seuil de référence pour une longueur d'onde déterminée précise, dite de référence $\lambda_f$, ce qui permettait d'effectuer une analyse qualitative et quantitative sans avoir à tracer les courbes, (en évitant donc d'avoir à utiliser un spectrographe), en se contentant de relever quelques points de mesure pour des longueurs d'onde précises significatives, après un calcul simple de mise à seuil.

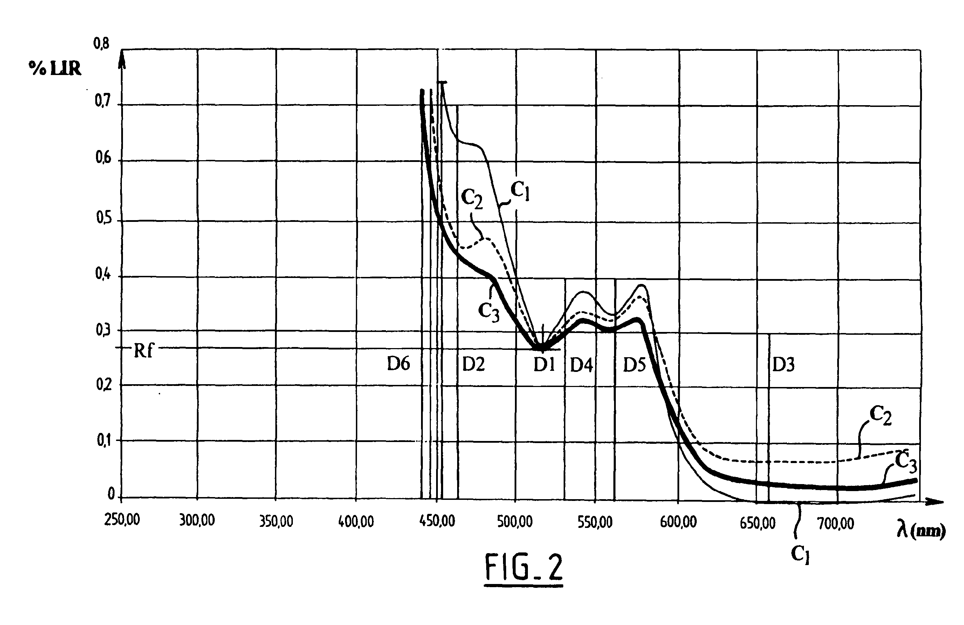

On se reportera maintenant à la figure 2 dans laquelle on a illustré trois courbes donnant en ordonnées l'absorption mesurée en % LIR en fonction de la longueur d'onde de la lumière reçue sur la peau de trois sujets différents. Cependant ces courbes ont été traitées mathématiquement de façon à les normaliser en permettant simultanément de comprendre le fonctionnement de l'appareil et du procédé conformes à l'invention.

Pour obtenir les courbes de la figure 2, on opère en quatre étapes successives.

<u>Première étape.</u> On procède à une opération de calibrage en effectuant une mesure de la réflexion sur un « gris » ou « blanc » « standard » (par exemple de la poudre de sulfate de baryum compactée). Pour obtenir de bons résultats et s'affranchir en particulier des dérives possibles des diodes, dans le temps et/ou en fonction de la température ambiante d'utilisation, cette opération de calibrage/étalonnage est effectuée avant chaque mesure sur le « gris » ou « blanc » standard choisi.

Dans l'exemple illustré on a supposé que l'on effectuait six mesures successives pour des longueurs d'onde respectives : 520 nm, 460 nm, 660 nm, 545 nm, 575 nm, et 430 nm. Dans la pratique, ces longueurs d'onde précises peuvent être émises par des diodes électroluminescentes DEL de qualité appropriée.

L'appareil de mesure a été schématisé à la figure 3. Tel que schématisé, l'appareil 1 comprend des diodes électroluminescentes DEL qui envoient un faisceau de lumière par exemple légèrement conique convergent (comme schématisé par les flèches) sur la peau 2 du sujet à examiner. La lumière réfléchie sensiblement perpendiculairement à la peau du sujet est reçue par un détecteur 3 qui analyse l'intensité du rayonnement reçu. L'utilisation d'un faisceau conique est avantageuse à plusieurs titres :

elle permet de s'affranchir en grande partie de la réflexion spéculaire et elle permet de déterminer de façon précise une distance optimale de positionnement $\underline{d}$ de l'appareil 1 par rapport à la peau, en effectuant la mesure de la réflexion lorsque l'intensité lumineuse d'éclairement est sensiblement réduite à un point sur la peau 2. L'angle du cône est avantageusement compris entre 30° et 50°, par exemple de l'ordre de 45°.

Le détecteur de l'appareil de mesure enregistre une intensité du facteur de réflexion :

$I_{0D1}$ pour la diode D1,
$I_{0D2}$ pour la diode D2,
.....
$I_{0D6}$ pour la diode D6.

<u>Deuxième étape.</u> On procède à une opération de mesure de réflexion sur la peau que l'on veut tester.

Lors d'une mesure sur la peau de l'enfant «x », le détecteur de l'appareil de mesure enregistre une intensité du facteur de réflexion :

$I_{xD1}$ pour la diode D1,
$I_{xD2}$ pour la diode D2,
.....
$I_{xD6}$ pour la diode D6.

<u>Troisième étape.</u> On procède maintenant au traitement mathématique de « normalisation » de façon que, pour une longueur d'onde donnée de référence, en l'occurrence ici celle de la première diode D1 à 520 nm, toutes les courbes passent par le même point de taux d'absorption ou facteur de réflexion mesuré en ordonnées.

Le calculateur, qui est avantageusement un microprocesseur, effectue les calculs de normalisation suivants :

$(I_{xD1}/I_{0D1}) = R_{x1}$ pour la diode D1

$(I_{xD2}/I_{0D2}) = R_{x2}$ pour la diode D2

.....

$(I_{xD6}/I_{0D6}) = R_{x6}$ pour la diode D6

Ensuite, on procède de telle façon que, pour la longueur d'onde de référence significative choisie : $\lambda_f$, tous les facteurs de réflexion $R_{x1f}$ soient égaux à une valeur de référence Rf.

$$R_{x1f} = R_f \qquad \rightarrow \qquad k = R_f/R_{x1}$$

puis l'on multiplie par le facteur $\underline{k}$ correspondant les différents facteurs de réflexion $R_{xn}$ pour obtenir les facteurs normalisés $R_{xnf}$.

$R_{x2f} = k\, R_{x2}$

.....

$R_{x6f} = k\, R_{x6}$

Le fait d'amener pour cette longueur d'onde de référence tous les taux de réflexion au même niveau de seuil Rf permet alors une lecture directe du taux recherché, par exemple de bilirubine, par simple lecture du LIR normalisé correspondant.

Quatrième étape. À partir des facteurs de réflexion normalisés, le microprocesseur calcule les LIR :

$LIR_{x1} = Log\,(1/R_{x1f}) = Log\,(1/R_f) = $ Constante
$LIR_{x2} = Log\,(1/R_{x2f})$
.....
$LIR_{x6} = Log\,(1/R_{x6f})$

Les LIR ainsi établis permettent de comparer avec des courbes spectrales, dans l'exemple illustré , trois courbes correspondant à des facteurs de réflexion différents de trois sujets examinés. On observe également que dans l'exemple des courbes illustrées, les courbes entières ont été en fait obtenues à partir d'un spectrographe analysant de façon continue les longueurs d'onde dans la plage allant de 430 nm à 750 nm, et ce pour avoir une représentation plus exacte du tracé de ces courbes, mais qui en fait ne sont pas nécessaires pour les mesures d'analyse des différents constituants de la peau comme il va être expliqué plus en détail ci-après en référence aux trois exemples donnés de mesure.

En revenant à la figure 2, on observe que pour la longueur d'onde de 460 nm on peut relever sur les trois courbes respectivement C1, C2, C3 des taux d'absorption respectifs d'environ 0,75, 0,55 et 0,5 mesurés en % LIR normalisé. La simple mesure de ce taux d'absorption permet de façon surprenante, comme on a pu le constater, de déduire que pour le sujet de la courbe C2 le taux de bilirubine est normal, tandis que pour le sujet de la courbe C1, le taux de bilirubine est élevé, alors que pour le sujet de la courbe C3, le taux de bilirubine est trop faible. Ces données (ces pourcentages de LIR) peuvent être simplement enregistrées dans une table de valeurs de référence préalablement expérimentées et connues.

De la même façon, il est possible d'apprécier la teneur en hémoglobine par le niveau des taux de réflexion pour la lumière émise par les diodes D4 à 545 nm ou 550 nm et D5 à 575 nm. Des mesures du taux de réflexion dans la région d'émission de la diode D6 vers 430 nm permettraient d'affiner les résultats.

La pigmentation de la peau pourra s'apprécier par le niveau des taux de réflexion en regard de la lumière émise par la diode D3 vers 660 nm (dans une plage comprise entre 620 et 780 nm). En fait, on observe qu'au delà, la courbe du LIR est pratiquement une droite, de sorte que la détermination de deux points de cette courbe suffisamment distants à partir de cette longueur d'onde, par exemple vers 620 nm et 780 nm permettront de déterminer avec précision la caractéristique de pigmentation correspondante : type africain, européen, asiatique, etc.

Le fait qu'avec le procédé de l'invention il n'est pas nécessaire de tracer les courbes entières mais que l'on peut se contenter de mesures limitées pour des longueurs d'onde précises bien définies s'explique par le fait que l'on s'affranchit des fluctuations passagères et des couleurs ou pigmentations propres des diverses peaux par le processus de « normalisation » précédemment expliqué.

En outre, comme on n'exerce aucune pression mécanique sur le tissu examiné, la mesure n'est pas faussée, en particulier si l'on veut mesurer le taux d'hémoglobine de la peau d'un individu par une pression exercée sur la peau qui chasserait le sang de l'endroit de mesure.

Ainsi, si l'on veut mesurer le taux de bilirubine de la peau claire d'un bébé calme, le taux de bilirubine est apprécié directement à partir du $LIR_{x2}$ mesuré avec la diode D2, en effectuant l'opération $LIR_{x2}$ - $LIR_{x1}$.

Si l'on mesure le taux de bilirubine sur une peau mate ou colorée (ethnie non européenne) et/ou d'un bébé agité (afflux de sang au niveau de la peau), les indications de $LIR_{x2}$ sont corrigées automatiquement de la même façon du fait de l'opération de soustraction $LIR_{x2}$ - $LIR_{x1}$, et du fait que la valeur $LIR_{x1}$ a été normalisée par l'opération précédemment décrite (même seuil Rf de réflexion normalisé pour la diode D1, et étalonné à chaque mesure).

En se reportant aux figures 4 à 6, on a indiqué comment du fait de l'utilisation d'un faisceau conique d'éclairement, il était immédiatement possible, comme illustré la figure 5, de déterminer la bonne distance de mesure d entre l'appareil et la peau du sujet. Lorsque l'appareil est trop près, comme illustré la figure 4, il ne se forme pas au niveau de la peau une tache ponctuelle comme à la figure 5, et il en va de même si l'appareil est trop loin, comme illustré à la figure 6.

L'appareil peut être ainsi conçu que lorsqu'il est dirigé vers la peau, l'éclairement ne se déclenche que lorsque l'appareil est à bonne distance, c'est-à-dire lorsque le faisceau lumineux converge pratiquement ponctuellement sur la peau.

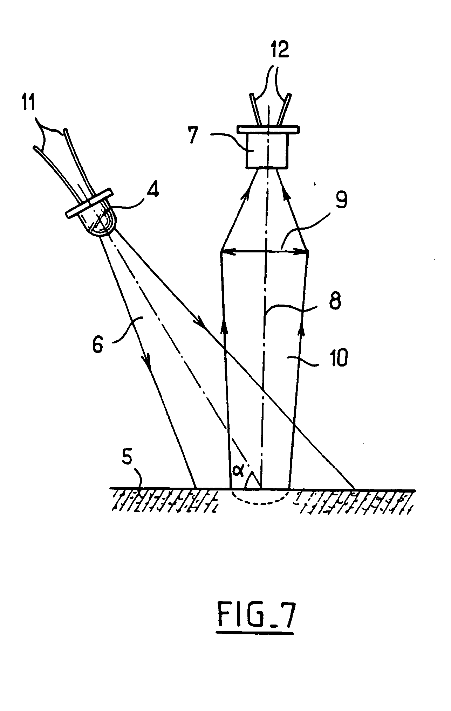

Selon la variante de réalisation illustrée à la figure 7, les diodes électroluminescentes DEL que comporte la tête de lecture, et qui sont schématisées en 4, éclairent la surface de la peau, schématisée en 5, sous un certain angle alpha ($\alpha$), avantageusement compris entre 30° et 60°, par exemple 45° ; le faisceau lumineux référencé 6 est réfléchi sur la peau ; en plaçant le capteur 7 de la tête de lecture du dispositif dans l'axe 8 perpendiculaire à la surface 5 de la zone éclairée de la peau, seule la partie de la lumière provenant de la diode électroluminescente 4 qui est diffusée par la peau est détectée par le capteur 7, et non la lumière réfléchie à la surface de la peau et qui dépend de la brillance de la peau. Il est ainsi possible d'obtenir une information plus fiable relativement à la caractéristique que l'on veut mesurer : taux de bilirubine, taux d'hémoglobine, etc..

Dans le schéma de la figure 7, une lentille 9 a été interposée sur le trajet du faisceau 10 de lumière diffusée par la peau de façon à augmenter la puissance lumineuse détecté par le capteur 7. En 11 et 12 ont été respectivement schématisés les fils d'alimentation des diodes électroluminescentes 4 et de sortie des signaux provenant du capteur 7.

De façon préférentielle, si l'on utilise cinq ou six diodes électroluminescentes DEL émettant des longueurs d'onde appropriées, telles que définies précédemment, on prévoira avantageusement que les diodes sont successivement alimentées de façon que le détecteur puisse successivement effectuer les mesures des taux de réflexion nécessaires à l'analyse des résultats. Préalablement à chaque mesure réalisée sur un tissu à analyser, l'appareil aura été calibré, comme mentionné précédemment, sur un « gris » ou « blanc standard » comme indiqué précédemment, et qui permettra de calculer le coefficient k explicité ci-dessus.

Bien que l'invention ait été décrite plus précisément en relation avec l'analyse du taux de bilirubine contenu dans la peau, ou d'autres constituants de la peau tels que la pigmentation, l'hémoglobine, etc., le principe de l'invention peut être étendu à l'analyse de tout tissu, à partir du moment où l'on déterminera quelle est ou quelles sont les longueurs d'onde précises pour laquelle ou lesquelles il faut relever le taux de réflexion pour avoir une mesure significative, et quelle est la longueur d'onde sur laquelle on calera toutes les courbes pour obtenir la normalisation desdites courbes.

**Revendications**

**1.** Procédé de mesure du taux d'un constituant d'un tissu, comportant les étapes selon lesquelles :

a/ on effectue la mesure d'une réflexion de la lumière d'une première longueur d'onde donnée et la mesure d'une réflexion de la lumière d'une seconde autre longueur d'onde déterminée servant de référence, sur un étalon standard;

b/ a/ on effectue la mesure d'une réflexion de la lumière de la première longueur d'onde donnée représentative de la mesure à effectuer et au moins la mesure d'une réflexion de la lumière de la seconde autre longueur d'onde déterminée servant de préférence, sur le tissu;

c/ on détermine un facteur de réflexion égal au rapport entre la mesure sur le tissu et la mesure sur l'étalon standard pour chacune de la première et de la deuxième longueur d'onde;

d/ on normalise les valeurs des dits facteurs de réflexion en ce que la valeur du facteur de réflexion pour la seconde longueur d'onde servant de référence soit égale à une valeur de référence choisie et:

- en calculant le rapport k de ladite valeur de référence choisie et du facteur de réflexion de cette seconde longueur d'onde;
- ledit facteur de réflexion de la première longueur d'onde en multipliant par le rapport k calculé,

e/on déduit le taux du constituant recherché en fonction d'une table de valeurs prédéterminées connues pour cette même première longueur d'onde et de ladite valeur du logarithme népérien de l'inverse du facteur de réflexion normalisé de la première longueur d'onde.

2. Procédé selon la revendication 1, selon lequel on effectue les étapes a) à e) sur un tissu d'un premier sujet pour déduire le taux du constituant recherché pour le premier sujet et on effectue les étapes a) à e): sur un tissu d'un deuxième sujet pour déduire le taux du constituant recherché pour le deuxième sujet, selon lequel on utilise pour les premier et second tissus la même première longueur d'onde donnée, la même seconde longueur d'onde déterminée servant de référence, et la même réflexion mesurée sur un étalon standard pour la deuxième longueur d'onde, selon lequel la normalisation effectuée permet de déterminer une même valeur du facteur réflexion normalisé pour la seconde longueur d'onde pour le premier sujet et pour le second sujet.

3. Procédé selon la revendication 1 ou 2, **caractérisé en ce que**, s'agissant de la peau d'un individu, on choisit comme seconde longueur d'onde : $\lambda_f = 520$ nm.

4. Procédé selon la revendication 3, **caractérisé en ce que** s'agissant de mesurer la bilirubine contenue dans la peau, on choisit comme longueur d'onde : $\lambda = 460$ nm.

5. Dispositif pour la mesure du taux d'un constituant d'un tissu, en particulier du taux de bilirubine dans la peau, comprenant:

   - une tête de lecture susceptible d'envoyer successivement plusieurs éclairs de longueurs d'onde diverses définies en direction du tissu à examiner et de recevoir et mesurer en retour la lumière réfléchie,
   - un calculateur, comprenant des moyens adaptés pour mettre en oeuvre le procédé selon l'une des revendications 1à 4.

6. Dispositif selon la revendication 5, **caractérisé en ce qu'**il comprend en tant qu'éléments pour envoyer séquentiellement des éclairs de longueurs d'onde déterminée des diodes électroluminescentes.

7. Dispositif selon la revendication 5 ou la revendication 6, **caractérisé en ce qu'**il envoie la lumière, avantageusement sous un angle de 30 degrés à 60 degrés et par exemple sensiblement égal à 45 degrés.

8. Dispositif selon l'une quelconque des revendications 5 à 7, **caractérisé en ce que**, s'agissant de la peau d'un individu, il utilise en tant que longueur d'onde de la lumière pour mesurer le point de référence une longueur d'onde voisine de 520 nm.

9. Dispositif selon l'une quelconque des revendications 5 à 8, **caractérisé en ce qu'**il utilise en tant que longueur d'onde de la lumière pour mesurer le taux de bilirubine une longueur d'onde voisine de 460 nm.

10. Dispositif selon l'une quelconque des revendications 5 à 9, **caractérisé en ce qu'**il utilise en tant que longueur d'onde de la lumière pour mesurer la pigmentation de la peau une longueur d'onde comprise entre 620.et 780 nm.

11. Dispositif selon l'une quelconque des revendications 5 à 10, **caractérisé en ce qu'**il utilise en tant que longueur d'onde de la lumière pour mesurer l'hémoglobine deux longueurs d'onde proches de 545 et 575 nm, et/ou une longueur d'onde voisine de 550 nm ou une longueur d'onde voisine de 430 nm.

**Claims**

1. Method for measuring the proportion of a constituent in a tissue, comprising the following steps:

   a) the measure of a reflection of the light with a first given wavelength and the measure of a reflection of the light with a second determined wavelength, used as a reference, are carried out on a standard;
   b) the measure of a reflection of the light with the first given wavelength, representative of the measure to be carried out, and at least the measure of a reflection of the light with the second other determined wavelength, used as a reference, are carried out on the tissue;
   c) a reflection factor equal to the ratio between the measure on the tissue and the measure on the standard for each of the first and of the second wavelength is determined;
   d) the values of said reflection factors are standardized in that the value of the reflection factor for the second wavelength used as a reference is equal to a selected reference value and:

    - by calculating the ratio k of said selected reference value and of the reflection factor of this second wavelength;
    - by multiplying said reflection factor of the first wavelength by the calculated ratio k;

e) the proportion of the searched constituent is deduced from a table of known predetermined values for this first wavelength and from the value of the natural logarithm of the multiplicative inverse of the standardized reflection factor for the first wavelength.

2. Method according to claim 1, wherein the steps a) to e) are carried out on a tissue of a first subject for deducing the proportion of the searched constituent for the first subject and the steps a) to e) are carried out on a tissue of a second subject for deducing the proportion of the searched constituent for the second subject, wherein the same first given wavelength, the same second determined wavelength used as a reference, and the same reflection measured on a standard for the second wavelength are used for the first and second tissues, wherein the standardization carried out enables to determine an equal value of the standardized reflection factor for the second wavelength for the first subject and for the second subject.

3. Method according to claim 1 or 2, **characterized in that** a second wavelength $\lambda_f$ = 520 nm is chosen for the skin of an individual.

4. Method according to claim 3, **characterized in that** a wavelength $\lambda$ = 460 nm is chosen for measuring the bilirubin contained in the skin.

5. Device for measuring the proportion of a constituent in a tissue, notably the proportion of bilirubin in the skin, comprising:

    - a reading head able to send successively a number of flashes of light with various defined wavelengths in the direction of the tissue to be examined and to receive and measure in return the reflected light,
    - a computer comprising means adapted to implement the method according to any of the claims 1 to 4.

6. Device according to claim 5, **characterized in that** it comprises light-emitting diodes used as elements for sequentially sending flashes of light with determined wavelengths.

7. Device according to claim 5 or claim 6, **characterized in that** it sends the light, advantageously with an angle from 30 degrees to 60 degrees and for example approximately equal to 45 degrees.

8. Device according to any of the claims 5 to 7,
**characterized in that**, for the skin of an individual, a wavelength around 520 nm is used as the wavelength of the light for measuring the reference point.

9. Device according to any of the claims 5 to 8,
**characterized in that** a wavelength around 460 nm is used as the wavelength of the light for measuring the proportion of bilirubin.

10. Device according to any of the claims 5 to 9,
**characterized in that** a wavelength between 620 et 780 nm is used as the wavelength of the light for measuring the skin pigmentation.

11. Device according to any of the claims 5 to 10,
**characterized in that** two wavelengths around 545 and 575 nm, and/or a wavelength around 550 nm or a wavelength around 430 nm, are used as a wavelength of the light for measuring hemoglobin.

**Patentansprüche**

1. Verfahren zur Messung der Proportion eines Bestandteils in einem Gewebe, umfassend die folgenden Schritte,

a) wobei die Messung einer Reflexion des Lichts mit einer ersten gegebenen Wellenlänge und die Messung einer Reflexion des Lichts mit einer zweiten bestimmten Wellenlänge, als Referenz, auf einem Standard durchgeführt werden;

b) wobei die Messung einer Reflexion des Lichts mit der ersten gegebenen Wellenlänge, repräsentativ für die durchzuführende Messung, und mindestens die Messung einer Reflexion des Lichts mit einer zweiten bestimmten Wellenlänge, als Referenz, auf dem Gewebe durchgeführt werden;

c) wobei ein Reflexionsgrad, gleich dem Verhältnis zwischen der Messung auf dem Gewebe und der Messung auf dem Standard für jede der ersten und der zweiten Wellenlänge bestimmt wird;

d) wobei die Werte der genannten Reflexionsgrade **dadurch** normalisiert werden, dass der Wert des Reflexionsgrads für die zweite Wellenlänge, als Referenz, gleich einem ausgewählten Referenzwert ist, und:

- indem das Verhältnis k des genannten Referenzwerts und des Reflexionsgrads dieser zweiten Wellenlänge berechnet wird;
- indem der genannte Reflexionsgrad der ersten Wellenlänge mit dem berechneten Verhältnis k multipliziert wird;

e) und wobei die Proportion des gesuchten Bestandteils aus der Tabelle von bekannten vorbestimmten Werten für diese erste Wellenlänge und aus dem Wert des natürlichen Logarithmus des Kehrwerts des normalisierten Reflexionsgrads für die erste Wellenlänge hergeleitet wird.

2. Verfahren nach Anspruch 1, wobei die Schritte a) bis e) auf einem Gewebe einer ersten Person durchgeführt werden, um daraus die Proportion des gesuchten Bestandteils für diese erste Person herzuleiten, und die Schritte a) bis e) auf einem Gewebe einer zweiten Person durchgeführt werden, um daraus die Proportion des gesuchten Bestandteils für diese zweite Person herzuleiten, wobei für die ersten und zweiten Gewebe die selbe erste gegebene Wellenlänge, die selbe zweite bestimmte Wellenlänge als Referenz, und die selbe auf einem Standard gemessene Reflexion für die zweite Wellenlänge verwendet werden, wobei für die erste Person und die zweite Person die durchgeführte Normalisierung die Bestimmung eines selben Werts des normalisierten Reflexionsgrads für die zweite Wellenlänge erlaubt.

3. Verfahren nach Anspruch 1 oder 2, **dadurch gekennzeichnet, dass** für die Haut eines Einzelwesens eine zweite Wellenlänge $\lambda_f$ = 520 nm ausgewählt wird.

4. Verfahren nach Anspruch 3, **dadurch gekennzeichnet, dass** zur Messung von Bilirubin in der haut eine Wellenlänge $\lambda$ = 460 nm ausgewählt wird.

5. Vorrichtung zur Messung der Proportion eines Bestandteils in einem Gewebe, insbesondere die Proportion von Bilirubin in der Haut, mit:

- einem Lesekopf zur Sendung mehrerer Blitzlichten mit verschiedenen definierten Wellenlängen nacheinander in Richtung des zu untersuchenden Gewebes und zum Empfang und zur Messung des reflektierten Lichts,
- einem Rechner mit Mitteln zur Durchführung des Verfahrens nach einem der Ansprüchen 1 bis 4.

6. Vorrichtung nach Anspruch 5, **dadurch gekennzeichnet, dass** sie Lumineszenzdioden als Elemente zur Sendung von Blitzlichten mit bestimmten Wellenlängen nacheinander umfasst.

7. Vorrichtung nach Anspruch 5 oder Anspruch 6, **dadurch gekennzeichnet, dass** sie das Licht, vorzugsweise mit einem Winkel von 30 bis 60 Grad und zum Beispiel mit einem Winkel sendet, der ungefähr 45 Grad gleich ist.

8. Vorrichtung nach einem der Ansprüche 5 bis 7, **dadurch gekennzeichnet, dass** für die Haut eines Einzelwesens eine Wellenlänge von etwa 520 nm als Wellenlänge des Lichts zur Messung des Referenzpunkts verwendet wird.

9. Vorrichtung nach einem der Ansprüche 5 bis 8, **dadurch gekennzeichnet, dass** eine Wellenlänge von etwa 460 nm als Wellenlänge des Lichts zur Messung die Proportion von Bilirubin verwendet wird.

10. Vorrichtung nach einem der Ansprüche 5 bis 9, **dadurch gekennzeichnet, dass** eine Wellenlänge zwischen 620 und 780 nm als Wellenlänge des Lichts zur Messung der Hautpigmentation verwendet wird.

11. Vorrichtung nach einem der Ansprüche 5 bis 10, **dadurch gekennzeichnet, dass** zwei Wellenlängen von etwa 545 und 575 nm und/oder eine Wellenlänge von etwa 550 nm oder eine Wellenlänge von etwa 430 nm als Wellenlänge des Lichts zur Messung von Hämoglobin verwendet werden.

$I_0$     $I_0R_1$     $I_0T_1^2R_2$    $I_0T_1^2T_2^2R_3$    $I_0T_1^2T_2^2T_3^2R_4$

1

2

3

4

<u>FIG.1</u>

3

1

d

2

<u>FIG.3</u>

1

2

<u>FIG.4</u>

1

2

<u>FIG.5</u>

1

2

<u>FIG.6</u>

10

FIG. 2

FIG_7

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5645061 A

**Littérature non-brevet citée dans la description**

- **Dawson, Barker.** une étude théorique et expérimentale de l'absorption et de la diffusion de la lumière par la peau in vivo. *Phys. Med. Biol.,* 1980, vol. 25 (4), 695-709